# EUROPÄISCHE PATENTANMELDUNG

(11) **EP 3 495 014 A1**
(43) Veröffentlichungstag der Anmeldung: **12.06.2019**
(21) Anmeldenummer: 18210746.6
(22) Anmeldetag: 06.12.2018
(51) Int. Cl.: A61M 35/00, A61J 7/00, A61M 31/00

(54) **SPENDERKOPF UND ZUGEHÖRIGES GRIFFTEIL ZUR VERABREICHUNG VON FLIESSFÄHIGEN SUBSTANZEN AN PATIENTEN**

(30) Priorität: 06.12.2017 DE 102017129000
(71) Anmelder: Evolmed GmbH, 39100 Bozen (IT)
(72) Erfinder: VOLGGER, Mirjam, 39100 Bozen (IT); EGEBRECHT, Michaela, 39011 Lana (IT)
(74) Vertreter: Kehl, Ascherl, Liebhoff & Ettmayr Patentanwälte Partnerschaft mbB

(57) **Zusammenfassung**

Die vorliegende Anmeldung bezieht sich bevorzugt auf einen Spenderkopf (10, 12) zum Einbringen von fließfähigen Substanzen in einen Mund eines Lebewesens. Der Spenderkopf umfasst ein sich länglich erstreckender Grundkörper, wobei der Grundkörper in Längserstreckungsrichtung (L) einerseits ein erstes Ende (25) ausbildet und andererseits ein zweites Ende (27) ausbildet, eine Kopplungsstelle (2) zum wiederholbaren und lösbaren Ankoppeln an einem Handteil (100), wobei die Kopplungsstelle (2) im Bereich (26) des ersten Endes (25) des Grundkörpers angeordnet oder ausgebildet ist, wobei der Grundkörper ein elastisch verformbares Material aufweist und elastisch verformbare Lamellen (20) ausbildet.

## Beschreibung

Die vorliegende Erfindung bezieht sich gemäß den Ansprüchen 1, 7 und 12 auf Spenderköpfe zum Einbringen von fließfähigen Substanzen in einen Mund eines Lebewesens, insbesondere eines Menschen, und gemäß Anspruch 14 auf einen Griff bzw. einen Griffteil, an dem ein solcher Spenderkopf angeordnet werden kann, und gemäß Anspruch 15 auf ein Set, das zumindest einen Spenderkopf und einen Griff aufweist.

### Hintergrund der Erfindung

Patienten mit Nahrungs- und Flüssigkeitsentzug in Folge einer Operation, aber auch lange Beatmungszeiten führen dazu, dass sich das physiologische Gleichgewicht der Mundschleimhäute verändert. Die Patienten leiden unter Mundtrockenheit, Rhagaden und Aften können die Folge sein. Diese Symptome sind unangenehm und schmerzhaft und können den gesamten

### Genesungsverlauf verzögern.

In der Krankenpflege werden Baumwollgazen auf eine hölzerne Zungenspatel gewickelt und in Flüssigkeiten wie Wasser, Himbeersaft o.ä. getränkt und dem Patienten gereicht oder der Mundraum damit befeuchtet. Die Patienten konnten dann die Flüssigkeit durch die Baumwollgaze (manchmal auch Watte) aufsaugen. Andernfalls mussten Substanzen mittels Hygienesticks (Stäbchen mit Schwämmchen) zur Anregung der Speichelproduktion verabreicht werden. Die Nachteile dieser Methode sind, dass sich der Baumwolltupfer von dem Holzspatel trennt und Textilfasern im Mund verbleiben. Zudem ist diese Methode ergonomisch für den Patienten nachteilig. Ferner sind speichelanregende Lösungen für die Patienten aufgrund des unnatürlich sauren Geschmacks unbeliebt.

### Aufgabe der vorliegenden Erfindung

Es ist die Aufgabe der vorliegenden Erfindung eine Vorrichtung zur verbesserten Einbringung von fließfähigen Substanzen in den Mund eines Patienten bereitzustellen. Bevorzugt soll dadurch eine Linderung bzw. Vermeidung von Mundtrockenheit, Pilzbefall und/oder Entzündungen in der Mundhöhle von Patienten bewirkt werden. Zusätzlich oder alternativ zur Versorgung mit Flüssigkeiten soll auch die Verabreichung von Nahrung und Wirkstoffen an den Patienten gewährleistet werden. Die Vorrichtung soll bevorzugt für bestimmte Zielgruppen anpassbar sein, beispielsweise von Arzneimittelherstellern als Applikator in Kombination bzw. befüllt mit Medikamenten und/oder Nährstoffen bereitstellbar sein.

### Beschreibung der Erfindung

Die zuvor genannte Aufgabe wird durch einen Spenderkopf zum Einbringen von fließfähigen Substanzen in einen Mund eines Lebewesens gemäß Anspruch 1 gelöst. Der erfindungsgemäße Spenderkopf weist dabei bevorzugt mindestens einen sich länglich erstreckenden Grundkörper auf, wobei der Grundkörper in Längserstreckungsrichtung einerseits ein erstes Ende ausbildet und andererseits ein zweites Ende ausbildet. Weiterhin weist der Spenderkopf eine Kopplungsstelle zum wiederholbaren und lösbaren Ankoppeln an einem Handteil bzw. einem Griff oder Griffteil auf, wobei die Kopplungsstelle im Bereich eines ersten Endes des Grundkörpers angeordnet oder ausgebildet ist. Der Grundkörper weist bevorzugt ein elastisch verformbares Material auf und bildet bevorzugt elastisch verformbare Lamellen aus. Die Lamellen weisen dabei bevorzugt eine mittlere Höhe zwischen 1 mm und 20 mm, insbesondere zwischen 2mm und 15mm, auf und sind bevorzugt zumindest abschnittsweise elastisch verformbar, insbesondere mehr als 2mm oder mehr als 3mm elastisch auslenkbar. Die Höhe der Lamellen gibt dabei die Erstreckungslänge der Lamelle von einer Übergangsstelle in einen den Innenraum des Grundkörpers begrenzenden Wandung hin zu einem losen Ende der Lamelle an, wobei dies bevorzugt in einem Querschnitt rechtwinklig zur Längserstreckungsrichtung des Grundkörpers bestimmt wird.

Diese Lösung ist vorteilhaft, da durch die Kopplungsstelle der Spenderkopf kraft- und/oder reibschlüssig an einem Griffteil ankoppelbar und lösbar ist. Ferner kann mittels der Lamellen eine fließfähige Substanz in den Mund eingebracht werden. Zusätzlich oder alternativ kann der Mundraum mittels der Lamellen behandelt, insbesondere massiert, werden.

Weitere bevorzugte Ausführungsformen der vorliegenden Erfindung sind Gegenstand der nachfolgenden Beschreibungsteile und/oder der Unteransprüche.

Gemäß einer weiteren bevorzugten Ausführungsform der vorliegenden Erfindung bilden die Lamellen mehr als 50%, insbesondere mehr als 60% oder mehr als 70% oder mehr als 80%, der Gesamtoberfläche des Grundkörpers aus. D.h., dass die äußere Oberfläche des Spenderkopfes bevorzugt größer ist als die innenliegende Oberfläche des Spenderkopfes. Diese Ausführungsform ist vorteilhaft, da die Lamellen zur Aufnahme großer Mengen fließfähiger Substanzen geeignet sind.

Bevorzugt weist der Spenderkopf mindestens oder genau zwei oder vier, insbesondere oder mindestens oder genau oder bis zu 6 oder mindestens oder genau oder bis zu 8 oder mindestens oder genau oder bis zu 10 oder mindestens oder genau oder bis zu 15, Lamellen auf. Die Lamellen weisen bevorzugt eine Dicke von mindestens 0,1mm, insbesondere von mindestens oder genau 0,2mm oder mindestens oder genau 0,5mm, und/oder eine Dicke von maximal oder genau 1mm oder von maximal oder genau 2mm oder von maximal oder genau 3mm oder von maximal oder genau 5mm auf.

Zusätzlich oder alternativ verändert sich die Höhe mindestens einer Lamelle in Abhängigkeit vom Abstand zum zweiten Ende des Grundkörpers. Bevorzugt nimmt die Höhe der Lamelle/n mit zunehmendem Abstand zum zweiten Ende zu.

Die Lamellen erstrecken sich gemäß einer weiteren bevorzugten Ausführungsform der vorliegenden Erfindung in ihrer Längserstreckungsrichtung zwischen dem ersten Ende und dem zweiten Ende des Grundkörpers. Durch die Lamellen wird bevorzugt ein erster Bereich, der im Bereich des ersten Endes ausgebildet ist, mit einem zweiten Bereich, der im Bereich des zweiten Endes ausgebildet ist, verbunden. Der erste Bereich erstreckt sich dabei um bis zu 45%, insbesondere bis zu 30% oder bis zu 20%, der Länge des Grundkörpers vom ersten Ende aus in Längsrichtung des Grundkörpers zum zweiten Ende hin. Der zweite Bereich erstreckt sich dabei um bis zu 30%, insbesondere bis zu 20% oder bis zu 15% oder bis zu 10% oder bis zu 5%, der Länge des Grundkörpers vom zweiten Ende aus in Längsrichtung des Grundkörpers zum ersten Ende hin. Diese Ausführungsform ist vorteilhaft, da eine zwischen den Lamellen aufgenommene fließfähige Substanz entlang der Lamellen in den Mund des Patienten fließen kann. Somit können relativ große Mengen an fließfähiger Substanz mit geringem Aufwand in den Mund des Lebewesens, insbesondere Menschen, eingebracht werden. Ferner kann mittels der Lamellen eine fließfähige Substanz in den Mund eingebracht werden. Dies dient einerseits der Versorgung mit Flüssigkeit, Nahrungsmitteln oder Medizin, andererseits zum Benetzen der Mundschleimhäute. Zusätzlich oder alternativ kann der Mundraum mittels der Lamellen behandelt, insbesondere gereinigt und massiert, werden.

Mehrere Lamellen oder alle Lamellen erstrecken sich gemäß einer weiteren bevorzugten Ausführungsform der vorliegenden Erfindung zumindest abschnittsweise und bevorzugt mehrheitlich oder vollständig spiralförmig, wobei im Kontext der vorliegenden Erfindung als spiralförmig auch helixförmig verstanden werden kann. Diese Ausführungsform ist vorteilhaft, da durch die Spiralform die Gesamtlänge der Lamellen länger ausgebildet werden kann als bei sich geradlinig erstreckenden Lamellen. Ferner wird durch die Spiralform eine Stützfunktion bewirkt, wodurch die Fließgeschwindigkeit der fließfähigen Substanz gegenüber sich gerade erstreckenden Lamellen reduziert wird.

Die Lamellen sind gemäß einer weiteren bevorzugten Ausführungsform der vorliegenden Erfindung in einem Winkel zwischen 45° und 135°, insbesondere von 90° oder im Wesentlichen 90° oder genau 90°, gegenüber der Längserstreckungsrichtung des Grundkörpers ausgerichtet. Diese Ausführungsform ist vorteilhaft, da die Fließgeschwindigkeit der fließfähigen Substanz deutlich reduziert wird. Ferner können die Lamellen zum Stimulieren der Mundbestandteile verwendet werden.

Die Lamellen sind scheibenförmig oder schalenförmig ausgebildet und weisen bevorzugt eine umlaufende Außenkante auf, die abschnittsweise oder vollständig eine runde oder elliptische Linie beschreibt, wobei die umlaufende Außenkante bei zumindest einer ersten Lamelle größer ist, als bei einer zweiten Lamelle, wobei die erste Lamelle näher zum ersten Ende hin angeordnet ist als zum zweiten Ende und die zweite Lamelle näher zum zweiten Ende hin angeordnet ist als zum ersten Ende. Bevorzugt sind mehrere Lamellen, insbesondere mehr oder mindestens oder bis zu 2, mehr oder mindestens oder bis zu 4, mehr oder mindestens oder bis zu 6, mehr oder mindestens oder bis zu 10, mehr oder mindestens oder bis zu 15, Lamellen vorgesehen. Besonders bevorzugt bilden zumindest zwei und bevorzugt die Mehrzahl oder alle Lamellen in Abhängigkeit Ihrer Position, insbesondere in Abhängigkeit des Abstands zum zweiten Ende, unterschiedliche Höhen gegenüber einer den Innenraum des Grundkörpers begrenzenden Wandung aus oder die umlaufende Außenkante der Lamellen ist verschieden. Es ist jedoch ebenfalls denkbar, dass mehrere Lamellen dieselben Abmessungen, insbesondere denselben Umfang, ausbilden. Bevorzugt beträgt der Abstand zweier Lamellen in Umfangsrichtung des Spenderkopfes zwischen 0,1mm und 15mm, insbesondere zwischen 1mm und 10mm oder zwischen 2mm und 7mm.

Die oben genannte Aufgabe wird ebenfalls durch einen Spenderkopf gemäß Anspruch 7 zum Einbringen von fließfähigen Substanzen in einen Mund eines Lebewesens gelöst. Der erfindungsgemäße Spenderkopf weist bevorzugt mindestens einen sich länglich erstreckenden Grundkörper auf, wobei der Grundkörper in Längserstreckungsrichtung einerseits ein erstes Ende ausbildet und andererseits ein zweites Ende ausbildet. Ferner ist am Spenderkopf bevorzugt eine Kopplungsstelle zum wiederholbaren und lösbaren Ankoppeln an einem Handteil insbesondere Griffteil oder Griff, vorgesehen, wobei die Kopplungsstelle bevorzugt im Bereich eines ersten Endes des Grundkörpers angeordnet oder ausgebildet ist. Der Grundkörper weist bevorzugt ein elastisch verformbares Material, insbesondere Gummi oder Silikon, auf. Der Grundkörper erstreckt sich in Tiefenrichtung im Bereich des zweiten Endes gegenüber der Erstreckung in Tiefenrichtung im Bereich des ersten Endes bevorzugt weiter und bevorzugt im Bereich des zweiten Endes weist die Wandung des Grundkörpers eine homogene Dicke auf. Bevorzugt erstreckt sich der Grundkörper in Tiefenrichtung im Bereich des zweiten Endes gegenüber der Erstreckung in Tiefenrichtung im Bereich des ersten Endes mindestens oder genau um den Faktor 1,1, insbesondere um mindestens oder genau oder bis zu einem Faktor von 1,25 oder mindestens oder genau oder bis zu einem Faktor von 1,5 oder mindestens oder genau oder bis zu einem Faktor von 2 oder mindestens oder genau oder bis zu einem Faktor von 2,5, weiter. Homogen bedeutet hierbei bevorzugt, dass mehr als 60% oder mehr als 70% oder mehr als 80% oder mehr als 90% des Volumens des Grundkörpers durch Wandungsanteile in Dickenrichtung begrenzt werden, wobei die Abstände der Wandungsanteile dabei vom kleinsten Abstand zum größten Abstand um weniger als den Faktor 3, insbesondere weniger als den Faktor 2,5 oder weniger als den Faktor 2 oder weniger als den Faktor 2 oder weniger als den Faktor 1,5 oder weniger als den Faktor 1,25 oder weniger als den Faktor 1,1, voneinander verschieden sind.

Gemäß einer weiteren bevorzugten Ausführungsform der vorliegenden Erfindung bildet der Grundkörper einen Innenraum zum Aufnehmen von fließfähigen Substanzen, insbesondere Nahrung und/oder Medikamenten und/oder Getränken und/oder Gels, aus, wobei der Grundkörper im Bereich der Kopplungsstelle eine Zuführöffnung zum Zuführen der fließfähigen Substanzen in den Innenraum aufweist. Diese Ausführungsform ist vorteilhaft, da der Spenderkopf mit unterschiedlichen Substanzen befüllbar ist. Bevorzugt ist der Ausdehnungsfaktor in Breitenrichtung zwischen dem ersten Bereich und dem zweiten Bereich kleiner als in Tiefenrichtung, insbesondere um mindestens, genau oder bis zu 10% oder 20% oder 30% oder 40% oder 50% oder 75% kleiner.

Ferner kann bei einem angekoppelten Griffteil eine ebenfalls im Griffteil vorgehaltene fließfähige Substanz in den Spenderkopf eingebracht werden. Der Innenraum bildet dabei bevorzugt eine Innenoberfläche aus, wobei die Außenoberfläche mindestens um den Faktor 1,1 oder 1,2 oder 1,5 oder 2 oder 2,5 oder 3 oder 4 oder 5 größer ist als die Innenoberfläche. Der Grundkörper bildet zudem eine Außenoberfläche aus. Die Außenoberfläche ist bevorzugt größer als eine Gesamtsumme definierter Flächenanteile, wobei die definierten Flächenanteile durch die Summe von 2x maximale Höhe des Grundkörpers x maximale Breite des Grundkörpers plus 2x maximale Tiefe des Grundkörpers x maximale Höhe des Grundkörpers, insbesondere plus maximale Tiefe x maximale Breite, insbesondere plus 2 x maximale Tiefe x maximale Breite, definiert ist. Der Grundkörper weist besonders bevorzugt eine Vielfaches, insbesondere mindestens das 1,1-fache oder mindestens oder genau 1,3-fache oder mindestens oder genau 1,5-fache oder mindestens oder genau 2-fache oder mindestens oder genau 2,3-fache oder mindestens oder genau 2,5-fache oder mindestens oder genau 3-fache, der Gesamtsumme der definierten Flächenanteile auf.

In einer den Innenraum des Grundkörpers begrenzenden Wandung sind gemäß einer weiteren bevorzugten Ausführungsform der vorliegenden Erfindung mehrere, insbesondere mindestens 2 oder mindestens oder genau oder bis zu 5 oder mindesten oder genau oder bis zu 10 oder mindesten oder genau oder bis zu 20 oder mindesten oder genau oder bis zu 50, definierte Austrittsöffnungen angeordnet oder eingebracht oder vorgesehen. Diese Ausführungsform ist vorteilhaft, da durch die Vielzahl der Austrittsöffnungen die fließfähige Substanz ohne größere Kraftbeaufschlagungen in den Mundraum des Patienten fließen kann. Die einzelnen Öffnungen haben dabei Öffnungsflächen bzw. Öffnungsquerschnitte von mindestens oder bis zu oder genau 0,1mm² oder von mindestens oder bis zu oder genau 0,2mm² oder von mindestens oder bis zu oder genau 0,3mm² oder von mindestens oder bis zu oder genau 0,6mm² oder von mindestens oder bis zu oder genau 1mm² oder von mindestens oder bis zu oder genau 1,5mm² oder von mindestens oder bis zu oder genau 2mm² oder von mindestens oder bis zu oder genau 3mm² oder von mindestens oder bis zu oder genau 5mm².

Gemäß einer weiteren bevorzugten Ausführungsform der vorliegenden Erfindung weisen einzelne, mehrere, die Mehrzahl oder alle Austrittsöffnungen jeweils einen Verschlussteil auf, wobei der Verschlussteil infolge einer über die äußere Oberfläche einleitbaren Unterdruckbeaufschlagung und/oder infolge einer über die innere Oberfläche einleitbaren Überdruckbeaufschlagung die Austrittsöffnung öffnet. Der Verschlussteil kann hierbei klappenartig, insbesondere als in der Wandung des Grundkörpers ausgebildete Klappe, oder schlitzartig oder schlitzförmig oder als Schlitz, insbesondere durch zwei aneinander anliegende Oberflächen, ausgebildet sein. Bevorzugt öffnen sich die Austrittsöffnungen, wenn ein Flüssigkeitsdruck im Innenraum des Grundkörpers aufgebaut wird und dieser Flüssigkeitsdruck einen definierten Schwellwert übersteigt. Zusätzlich oder alternativ öffnen sich die Austrittsöffnungen, wenn ein Unterdruck auf den Grundkörper wirkt und dieser Unterdruck einen definierten Schwellwert übersteigt. Alternativ kann auch eine Schwellwertkombination aus auf die äußere Oberfläche des Grundkörpers aufgebrachten Unterdruck und aus im Innenraum erzeugten Flüssigkeitsdruck zum Öffnen der Austrittsöffnungen führen, wobei bei der Schwellwertkombination der Schwellwert für den Unterdruck und der Schwellwert für den Flüssigkeitsdruck bevorzugt unterhalb der Schwellwerte liegen, die bei nicht-kombinierter Beaufschlagung auftreten bzw. erforderlich sind. Im Falle von einem oder mehreren schlitzartigen oder schlitzförmigen oder als Schlitz ausgebildeten Verschlussteil/en werden die den Schlitz begrenzenden Wandungsanteile bzw. aneinander liegenden Oberflächen infolge der Unterdruckbeaufschlagung, die von außen auf den Spenderkopf wirkt und/oder infolge der Überdruckbeaufschlagung, die vom Inneren des Spenderkopfs auf den Spenderkopf wirkt, geöffnet. Diese Ausführungsform ist vorteilhaft, da bei verschlossenen Austrittsöffnungen ein Transport des befüllten Spenderkopfes möglich ist, ohne dass durch die Austrittsöffnungen die fließfähige Substanz austritt. Dies ist insbesondere unmittelbar vor der Verabreichung der fließfähigen Substanz an den Patienten sinnvoll, da dadurch die fließfähige Substanz, insbesondere bei geringer Zähflüssigkeit, wie z.B. bei Wasser, Tee, Saft, etc., nicht auf den Patienten tropft.

Im Innenraum des Spenderkopfes ist gemäß einer weiteren bevorzugten Ausführungsform eine saugfähige Einrichtung bzw. ein saugfähiges Element, insbesondere ein Schwamm oder ein Textil, insbesondere auswechselbar, angeordnet. Diese Ausführungsform ist vorteilhaft, da durch die saugfähige Einrichtung ein ungewolltes Abtropfen der fließfähigen Substanz weiter begrenzt wird. Zusätzlich oder alternativ kann die äußere Oberfläche des Grundkörpers eine Vielzahl an Erhebungen und/oder Vertiefungen, insbesondere Noppen und/oder Rillen, insbesondere Längsrillen und/oder Querrillen, und/oder Lamellen, insbesondere Längslamellen und/oder Querlamellen, zum Reinigen der Zunge und/oder der Schleimhäute aufweisen. Diese Ausführungsform ist vorteilhaft, da der Spenderkopf weitere Stimulationen des Mundraums ermöglicht. Als Vielzahl können hierbei insbesondere mindestens 2 oder mindesten oder genau oder bis zu 5 oder mindesten oder genau oder bis zu 10 oder mindesten oder genau oder bis zu 20 oder mindesten oder genau oder bis zu 50 oder mindesten oder genau oder bis zu 100, definierte Erhebungen und/oder Vertiefungen verstanden werden. Die Noppen und/oder Querrillen und/oder Querlamellen sind dabei bevorzugt einerseits oder beiderseits oder vollumfänglich an dem Grundkörper angeordnet. Die Noppen und/oder Querrillen und/oder Querlamellen sind bevorzugt im Bereich des zweiten Endes ausgebildet.

Die zuvor genannten Spenderköpfe und/oder das Griffteil werden bevorzugt mittels eines Spritzgussverfahrens erzeugt.

Die zuvor genannte Aufgabe wird ferner gemäß Anspruch 11 durch einen Spenderkopf zum Einbringen von fließfähigen Substanzen in einen Mund eines Lebewesens gelöst. Dieser Spenderkopf weist bevorzugt mindestens einen sich länglich erstreckenden Grundkörper auf, wobei der Grundkörper in Längserstreckungsrichtung einerseits ein erstes Ende ausbildet und andererseits ein zweites Ende ausbildet. Ferner weist der Spenderkopf bevorzugt eine Kopplungsstelle zum wiederholbaren und lösbaren Ankoppeln an einem Handteil oder einem Griffteil auf. Die Kopplungsstelle ist dabei bevorzugt im Bereich eines ersten Endes des Grundkörpers angeordnet oder ausgebildet. Der Grundkörper weist bevorzugt ein elastisch verformbares Material auf, wobei der Grundkörper durch einen definiert geformten Schwamm, insbesondere einen Konjakschwamm, ausgebildet wird, wobei die Kopplungsstelle zur definierten reib- und/oder kraftschlüssigen Kopplung mit einem Verbindungsteil, insbesondere eines Griffteils, aufbereitet ist. Diese Lösung ist vorteilhaft, da der bevorzugt einstückige Spenderkopf zum einen definiert fixierbar ist und zum anderen aufgrund des Materials sehr angenehm für die Patienten ist. Ferner weist der Spenderkopf bevorzugt einen Innenraum auf. Der Innenraum wird dabei bevorzugt mittels eines Schneidwerkzeugs oder eines spanenden Werkzeugs oder eines brennenden bzw. glimmenden bzw. heißen, insbesondere mehr als 70°C oder mehr als 100°C oder mehr als 150°C heißen, Werkzeugs erzeugt. Bevorzugt ist der Innenraum kommunizierend mit dem Innenraum eines Griffteils verbindbar. Der Innenraum weist bevorzugt eine Länge von mehr als 5mm, insbesondere von mehr als 10mm oder von mehr oder bis zu 15mm oder von mehr oder bis zu 25mm, auf. Weiterhin weist der Innenraum bevorzugt abschnittsweise einen Durchmesser von mindestens 3mm oder von mehr als oder bis zu 5mm oder von mehr als oder bis zu 10mm oder von mehr als oder bis zu 15mm oder von mehr als oder bis zu 25m auf. Der Durchmesser ist dabei bevorzugt ein über die Länge bestimmter mittlerer Durchmesser.

Im Bereich des ersten Endes des Grundkörpers ist gemäß einer weiteren bevorzugten Ausführungsform der vorliegenden Erfindung ein umlaufender Kragen ausgebildet, wobei sich der Kragen in radialer Richtung weiter erstreckt als alle anderen Bestandteile des Spenderkopfs.

Der Spenderkopf besteht bevorzugt aus einem Polymermaterial oder weist ein Polymermaterial auf. Das Polymermaterial ist bevorzugt Gummi oder Silikon oder weist bevorzugt Gummi oder Silikon auf. Der Spenderkopf ist bevorzugt einstückig, d.h. dass der Grundkörper und die daran ausgebildete Kopplungsstelle bevorzugt in einem Fertigungsschritt erzeugt werden und/oder eine gemeinsame Leitstützstruktur ausbilden. Wird ein saugfähiges Element in den durch den Grundkörper begrenzten Innenraum eingebracht, dann ist dieses saugfähige Element, insbesondere ein Schwamm oder ein Textil nicht einstückig mit dem Spenderkopf erzeugt.

Die hier beschriebenen Spenderköpfe können alternativ auch als Mundstücke bezeichnet werden. Bevorzugt weisen einzelne oder alle der beschriebenen Spenderköpfe einzelne oder mehrere oder die Mehrzahl oder alle der nachfolgenden Eigenschaften auf:
Bevorzugt stellen die Spenderköpfe aus hygienischen Gründen Einwegartikel dar. Die Spenderköpfe sind bevorzugt für unterschiedliche Zielgruppen / Anforderungen / Anwendungen (Befeuchtung von Mundschleimhäuten, Gabe von Medizin, Kühlen des Mundes, Gabe von püriertem Obst und Gemüse, etc.) geeignet. Bevorzugt weisen die Spenderköpfe ein durchlässiges Material zum Aufnehmen und/oder Abgeben der fließfähigen Substanz auf. Weiterhin sind die Spenderköpfe bevorzugt ergonomisch / angenehm im Mund, insbesondere weich. Weiterhin ist es erfindungsgemäß möglich, dass der Grundkörper einen oder mehrere Geschmacksstoffe, Wirkstoffe und/oder Nährstoffe aufweist oder daraus besteht und/oder damit beschichtet ist. Wobei der Wirkstoff, insbesondere eine Säure, wie z.B. Zitronensäure, bevorzugt zur Anregung der Speichelproduktion dient. Die Spenderköpfe können auch als austauschbare Kartusche, die bereits mit verschiedenen Inhaltsstoffen (z.B. Tabs mit klinischer Nahrung) vorbefüllt ist, bereitgestellt werden. Alternativ können die Spenderköpfe individuell befüllbar sein oder mit Arzneien befüllt werden (Applikator (Pharma)).

Anwendungsbereiche der Spenderköpfe sind hier z.B. die Onkologie, Intensivmedizin, Anästhesie, Post-OP, Kranken- und Altenpflege (z.B. Palliativbetreuung), Neurologie, Zahnmedizin.

Weiterhin können die Spenderköpfe bei Patienten aller Altersstufen mit unterschiedlichen Krankheitsbildern und Bedürfnissen eingesetzt werden.

Die vorliegende Erfindung bezieht sich gemäß Anspruch 14 ferner auf ein Griffteil zur Ankopplung an einen Spenderkopf gemäß einem der Ansprüche 1 bis 13. Der Griffteil weist dabei bevorzugt mindestens einen sich bevorzugt länglich erstreckenden Basiskörper auf. Der Basiskörper bildet dabei bevorzugt einen elastisch verformbaren Aufnahmeraum zum Aufnehmen einer Flüssigkeit und eine Öffnung zum Zuführen und Ausgeben der Flüssigkeit aus, wobei das Volumen des Aufnahmeraums infolge einer elastischen Verformung veränderbar ist. Die Festigkeit des Basiskörpers ist dabei an der schwächsten Stelle bzw. an der Stelle mit der geringsten Festigkeit bevorzugt zumindest oder genau um den Faktor 1,1 oder 1,2 oder 1,3 oder 1,5 oder 2 oder 2,5 oder 3 größer als die Festigkeit des Spenderkopfs, insbesondere an der stärksten Stelle bzw. an der Stelle mit der höchsten Festigkeit oder an der Stelle mit der geringsten Festigkeit. Die Öffnung des Griffs ist im Bereich einer Anbringungseinrichtung zur Ankopplung des Spenderkopfs ausgebildet. Die Anbringungseinrichtung ist zum, insbesondere kraftschlüssigen und/oder formschlüssigen, Zusammenwirken mit einem an einem Spenderkopf angeordneten Verbindungsteil oder einer Kopplungsstelle des Spenderkopfs ausgebildet. Der Griffteil weist bevorzugt einzelne oder mehrere oder die Mehrzahl oder alle der nachfolgenden Eigenschaften auf: Er ist wiederverwendbar, ergonomisch (für Pfleger und Patient) und/oder leicht zu reinigen / hygienisch.

Der Aufnahmeraum ist infolge einer elastischen Verformung verkleinerbar, wobei die den Aufnahmeraum begrenzende Wandung eine Rückstellkraft zum Überführen des Aufnahmeraums in eine vor der elastischen Verformung vorliegende Konfiguration vorhält bzw. erzeugt. Somit kann der Aufnahmeraum komprimiert werden und infolge einer Überführung des Aufnahmeraums in die Ausgangskonfiguration Flüssigkeit in den Aufnahmeraum eingesaugt werden. Dies ist bevorzugt ebenfalls im Falle eines an den Griffteil angekoppelten Spenderkopf möglich. Bevorzugt weist der Aufnahmeraum des Griffteils ein größeres Volumen oder das gleiche Volumen oder ein kleineres Volumen als der Innenraum des Spenderkopfes auf.

Die vorliegende Erfindung bezieht sich gemäß Anspruch 15 auf ein Set. Das Set besteht bevorzugt zumindest aus einem Griffteil nach Anspruch 14 und einem Spenderkopf nach einem der Ansprüche 1 bis 13 und bevorzugt aus einem Aufnahmebehältnis zum Vorhalten einer Flüssigkeit und zum Einbringen des Spenderkopfs. Der Aufnahmeraum des Griffteils und der Innenraum des Spenderkopfs bilden in einer Gebrauchskonfiguration bevorzugt eine kommunizierende Fluidverbindung aus.

Das Set kann zusätzlich oder alternativ ein modulares System von mehreren austauschbaren Spenderköpfen und einem oder mehreren wiederverwendbaren Griffteilen und/oder einer oder mehreren Verschlusseinrichtungen bestehen.

Das Aufnahmebehältnis kann auch als Kappe bezeichnet werden. Bevorzugt weist das Aufnahmebehältnis eine Aufnahme für den Griff auf und/oder bildet eine hygienische Abdeckung für den Spenderkopf aus und/oder kann als Gefäß zum Aufladen des Mundstücks mit einer fließfähigen Substanz, insbesondere einer Flüssigkeit, dienen.

Die Gabe von Tropfen, Gel, Sirup oder anderen Medikamenten "per os" bewirkt in der Mundschleimhaut eine schnelle Aufnahme des pharamakologischen Wirkstoffs und hat somit einen raschen therapeutischen Effekt. Eine gewünschte Medikamentendosis kann somit erfindungsgemäß über den Spenderkopf verabreicht werden. Hierdurch wird eine bessere Kontrolle erreicht, d.h. es wird leichter erfasst, dass wirklich die gesamte Dosis den Mundinnenraum erreicht, wodurch auch das Ziel der Wirksamkeit besser garantiert wird.

Weitere Vorteile, Ziele und Eigenschaften vorliegender Erfindung werden anhand nachfolgender Beschreibung anliegender Zeichnungen erläutert, in welchen beispielhaft erfindungsgemäße Griffteile und Spenderköpfe dargestellt sind. Elemente der erfindungsgemäßen Einrichtungen und Verfahren, welche in den Figuren wenigsten im Wesentlichen hinsichtlich ihrer Funktion übereinstimmen, können hierbei mit gleichen Bezugszeichen gekennzeichnet sein, wobei diese Bauteile bzw. Elemente nicht in allen Figuren beziffert oder erläutert sein müssen. Nachfolgend wird die Erfindung rein beispielhaft anhand der beigefügten Figuren beschrieben. Darin zeigt:
- Fig. 1a-1e: Darstellungen einer ersten exemplarischen Ausführungsform des erfindungsgemäßen Spenderkopfes mit zugehörigem Griffteil;
- Fig. 2: eine Darstellung einer zweiten exemplarischen Ausführungsform;
- Fig. 3a-3e: Darstellungen einer dritten exemplarischen Ausführungsform des erfindungsgemäßen Spenderkopfes mit zugehörigem Griffteil;
- Fig. 4: eine Detaildarstellung eines Bestandteils des Spenderkopfes gemäß der dritten exemplarischen Ausführungsform;
- Fig. 5a-5e: Darstellungen einer vierten exemplarischen Ausführungsform des erfindungsgemäßen Spenderkopfes mit zugehörigem Griffteil;
- Fig. 6a-6d: Darstellungen eines exemplarischen Griffteils; und
- Fig. 7a-7b: Darstellungen eines Sets bestehend aus Kappe, Griffteil und Spenderkopf.

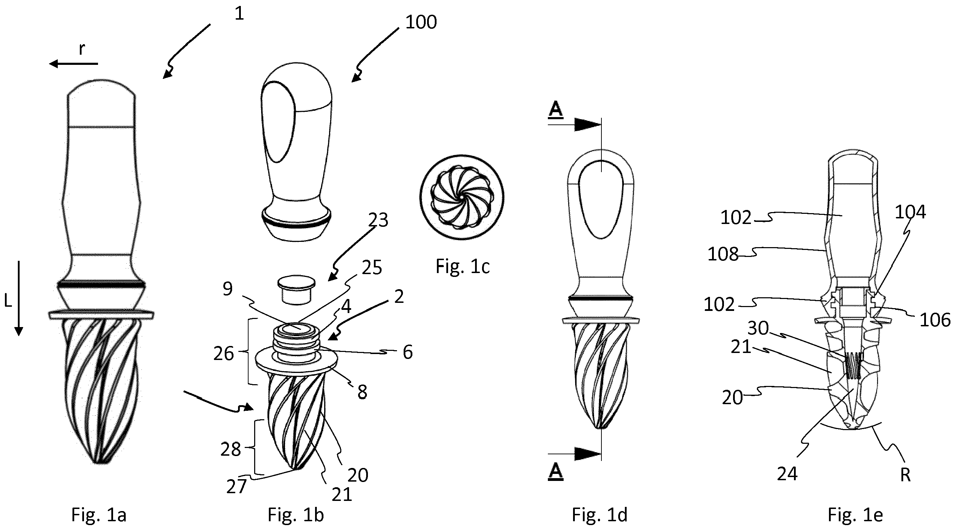

Fig. 1a zeigt eine erfindungsgemäße Spendervorrichtung 1. Die Spendervorrichtung 1 weist dabei bevorzugt zumindest einen Griffteil 100 und einen Spenderkopf 10, insbesondere mit spiralförmigen Lamellen 20, auf.

Gemäß Fig. 1b kann zwischen dem Griffteil 100 und dem Spenderkopf 10 eine Verschlusseinrichtung 22, insbesondere ein Deckel oder ein Blättchen, wie z.B. eine Kunststoffscheibe, zur Verhinderung oder Begrenzung eines Fluidtransfers zwischen einem Innenraum 122 des Griffteils 100 und einem Innenraum bzw. Aufnahmeraum 24 des Spenderkopfes 10 (vgl. Fig. 1e) vorgesehen bzw. angeordnet sein.

Der Spenderkopf 10 weist bevorzugt mehr als vier Lamellen 20 auf, wobei die Lamellen 20 bevorzugt um mindestens oder bis zu oder genau 5° oder 10° oder 15° oder 30° oder 45° oder 60° oder 75° oder 90° oder 105° oder 120° oder 135° oder 140° um die Längsachse L des Spenderkopfes 10 rotieren bzw. erstrecken. Es ist hierbei möglich, dass die Steigung der durch die Lamellen 20 beschriebenen Kurven konstant oder veränderlich ist. Bevorzugt ist die Steigung im Bereich 28 eines zweiten in Längsrichtung L ausgebildeten Endes 27 von der Steigung im Bereich 26 des in Längsrichtung L ausgebildeten ersten Endes 25 verschieden. Die einzelnen Lamellen 20 bilden beiderseits jeweils Oberflächenanteile 21 aus, die gegenüber von Oberflächenanteilen 21 anderer Lamellen 20 liegen. Im Bereich 28 des zweiten Endes 27 bildet der Spenderkopf 10 bevorzugt eine Spitze aus. Diese Spitze (im Querschnitt - vgl. Fig. 1e) kann abgerundet ausgebildet sein, insbesondere z.B. durch einen Radius R definiert sein, der oder genau oder bis zu oder größer ist als 2cm oder genau oder bis zu oder größer ist als 3cm oder genau oder bis zu oder größer ist als 5cm oder genau oder bis zu oder größer ist als 10cm oder genau oder bis zu oder größer ist als 15cm oder genau oder bis zu oder größer ist als 20cm oder genau oder bis zu oder größer ist als 25 oder genau oder bis zu oder größer ist als 30cm.

Der Spenderkopf 10 bildet in Bereich 28 des zweiten Endes 27 ausgehend von der Längsachse eine mittlere radiale Erstreckung aus, die geringer ist als die mittlere radiale Erstreckung im Bereich 26 des ersten Endes 25. Bevorzugt ist die mittlere radiale Erstreckung im Bereich 28 des zweiten Endes 27 zumindest oder genau um den Faktor 1,1 oder zumindest oder genau um den Faktor 1,5 oder zumindest oder genau um den Faktor 2 oder zumindest oder genau um den Faktor 2 oder zumindest oder genau oder bis zu dem Faktor 2,5 größer als die mittlere radiale Erstreckung im Bereich 26 des ersten Endes 25.

Im Bereich 26 des ersten Endes 25 ist eine Öffnung 9 zur Einbringung von fließfähigen Substanzen in den Innenraum 24 des Spenderkopfes 10 ausgebildet. Die Öffnung 9 wird dabei bevorzugt durch eine Kopplungsstelle 2 in Umfangsrichtung begrenzt. Die Kopplungsstelle 2 weist bevorzugt ein oder mehrere, insbesondere mindestens 2 oder genau 2 oder mehr als 3 oder genau 3 oder bis zu 3 Fixierelemente 4, 6 zum formschlüssigen und/oder kraftschlüssigen Ankoppeln an ein Griffteil 100 auf. Das oder die Fixierelemente 4, 6 können dabei ein Gewinde oder ein Bajonettverschluss oder eine oder mehrere Elemente zur Erzeugung einer rein formschlüssigen Verbindung mit dem Griffteil 100 ausbilden. Im gezeigten Beispiel sind die Fixierelemente 4, 6 als ein erstes Formschlusselement 4 und als ein zweites Formschlusselement 6 ausgebildet. Es ist hierbei möglich, dass nur eines des Formschlusselemente 4, 6 vorgesehen ist. Die Formschlusselemente 4, 6 sind hierbei bevorzugt als umlaufende Krägen oder Erhöhungen ausgebildet. Die Formschlusselemente 4, 6 bzw. der erste Bereich 26 ist bevorzugt flexibel, insbesondere elastisch verformbar, ausgebildet, wodurch die Formschlusselemente 4, 6 infolge einer Stauchung in ein Griffteil 100 eingeführt werden können und aufgrund der elastischen Rückverformung mit einer korrespondierenden Griffkopplungsstelle 102 bzw. Griffteilkopplungsstelle 102 zusammenwirken (vgl. Fig. 1e).

Fig. 1e zeigt ferner, dass die Griffkopplungsstelle 102 Gegenstücke 104, 106, insbesondere umlaufende Nuten 104, 106, zur Aufnahme der Formschlusselemente 4, 6 aufweist bzw. ausbildet. Die Griffkopplungsstelle 102 ist hierbei im Bereich einer Öffnung des Griffteils 100 angeordnet, insbesondere in einem Abstand zur Öffnung des Griffteils 100 der geringer ist als 3cm oder geringer oder gleich ist zu 2cm oder geringer oder gleich ist zu 1cm.

Weiterhin zeigt Fig. 1b, dass der Spenderkopf 10 bevorzugt einen Kragen 8 aufweist. Der Kragen 8 erstreckt sich dabei in radialer Richtung r weiter als die verbleibenden Anteile des Spenderkopfes 10. Dies hat den Vorteil, dass verhindert wird, dass eine von den Lamellen 20 aufgenommene fließfähige Substanz unmittelbar auf den Griffteil 100 überfließt. Der Kragen 8 bildet somit bevorzugt einen Fließstop bzw. eine Einrichtung zur Reduzierung der Fließgeschwindigkeit der fließfähigen Substanz aus.

Fig. 1c illustriert eine Draufsicht auf den erfindungsgemäßen Spenderkopf 10.

Fig. 1d kennzeichnet einen Schnitt A-A entlang der Spendervorrichtung 1 und durch Fig. 1e wird die sich aus dem Schnitt A-A ergebende Schnittdarstellung wiedergegeben.

Fig. 1e lässt sich ferner entnehmen, dass der Griffteil 100 bevorzugt einstückig durch eine Wandung 108 ausgebildet wird, wobei die Wandung 108 den Innenraum 122 begrenzt und die Griffkopplungsstelle 102 ausbildet. Der Griffteil 100 kann bevorzugt einstückig oder mehrstückig, insbesondere zweistückig, erzeugt werden. Besonders bevorzugt ist die Wandung 108 in dem den Innenraum 122 umschließenden Anteil abschnittsweise oder mehrheitlich elastisch verformbar. Dies ist vorteilhaft, da dadurch im Innenraum 122 vorgehaltene fließfähige Substanzen aus dem Innenraum 122 herauspressbar sind. Ferner kann infolge einer Rückverformung der zusammengedrückten Wandung 108 eine fließfähige Substanz in den Innenraum 122 des Griffteils 100 gesaugt werden, insbesondere über einen angekoppelten Spenderkopf 10 hinweg bzw. hindurch.

Das Bezugszeichen 30 kennzeichnet eine Austrittsöffnung, durch welche die fließfähige Substanz aus dem Aufnahmeraum 24 des Spenderkopfs 10 heraustreten kann. Bevorzugt weist der Spenderkopfs 10 eine Vielzahl an Austrittsöffnungen 30 auf, insbesondere mehr als vier oder mehr als 10 oder bis zu 10 oder mehr als 20 oder bis zu 20 Austrittsöffnungen 30. Die Austrittsöffnungen 30 können hierbei als Schlitze und/oder Löcher, insbesondere runde oder rechteckige oder ovale Löcher, ausgebildet sein.

Die vorliegende Erfindung bezieht sich somit bevorzugt auf einen Spenderkopf 10, 12 zum Einbringen von fließfähigen Substanzen in einen Mund eines Lebewesens. Der erfindungsgemäße Spenderkopf 10, 12 umfasst dabei bevorzugt ein sich länglich erstreckender Grundkörper, wobei der Grundkörper in Längserstreckungsrichtung L einerseits ein erstes Ende 25 ausbildet und andererseits ein zweites Ende 27 ausbildet, eine Kopplungsstelle 2 zum lösbaren Ankoppeln an einem Handteil 100, wobei die Kopplungsstelle 2 im Bereich 26 des ersten Endes 25 des Grundkörpers angeordnet oder ausgebildet ist. Bevorzugt weist der Grundkörper ein elastisch verformbares Material auf und bildet elastisch verformbare Lamellen 20 aus.

Die Austrittsöffnungen 30 können hierbei somit einen oder mehrere Verschlussteile aufweisen, die jeweils als Klappe und/oder als Schlitz, ausgebildet sind und in einer Öffnungskonfiguration Löcher bzw. Durchgangslöcher bzw. Durchgangsöffnungen darstellen, die bevorzugt eine runde oder rechteckige oder ovale oder längliche Kontur ausbilden.

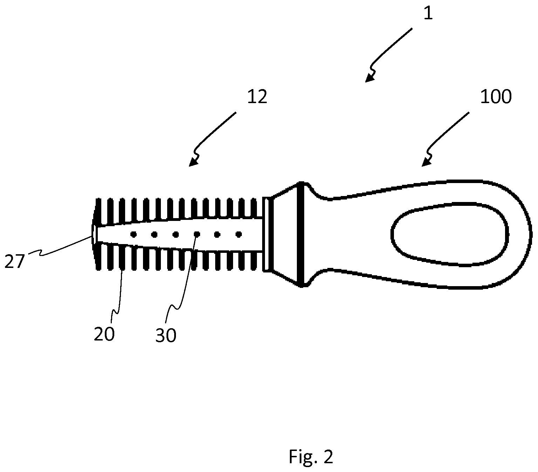

Fig. 2 zeigt ein weiteres Beispiel einer erfindungsgemäßen Spendervorrichtung 1. Diese Spendervorrichtung 1 weist dabei den beschriebenen Griffteil 100 und einen alternativ daran angekoppelten Spenderkopf 12 auf. Der Spenderkopf 12 weist eine Vielzahl in Längsrichtung L voneinander beabstandete Lamellen 20 auf. Die Lamellen 20 können sich dabei in radialer Richtung r gleich weit oder unterschiedlich weit erstrecken. Im gezeigten Beispiel erstrecken sich die Lamellen 20 im Wesentlichen in radialer Richtung r gleich weit. Die Lamellen 20 erstrecken sich dabei bevorzugt vollständig in Umfangsrichtung bzw. umschließen den Innenraum 24 bzw. einen den Innenraum 24 ausbildenden Grundkörper vollständig in Umfangsrichtung.

Es ist ebenfalls erfindungsgemäß möglich, dass sich die im Bereich 28 des zweiten Endes 27 angeordneten Lamellen 20 sich in radialer Richtung r weniger weit erstrecken als zum zweiten Ende 27 weiter beabstandete Lamellen 20.

Die Lamellen 20 weisen bevorzugt eine runde oder ovale Form auf. Bevorzugt sind die Lamellen 20 in gleichen oder verschiedenen Abständen zueinander angeordnet. Bevorzugt ist der Abstand zweier Lamellen 20 zueinander größer als die Dicke der jeweiligen Lamellen 20 oder kleiner als die Dicke der jeweiligen Lamellen oder entspricht der Dicke der jeweiligen Lamellen 20. Weiterhin weist der den Innenraum 24 begrenzende Grundkörper bevorzugt Austrittsöffnungen 30 auf.

Es ist hierbei jedoch bevorzugt stets möglich, dass durch die Austrittsöffnungen 30 auch fließfähige Substanz in den Innenraum 24 der erfindungsgemäßen Spenderköpfe 10, 12 einbringbar, insbesondere einsaugbar, ist.

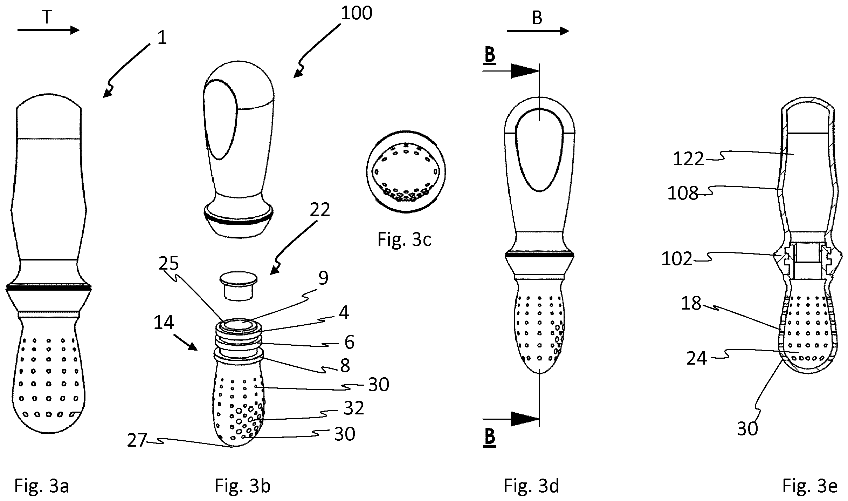

Fig. 3a zeigt ein weiteres Beispiel einer erfindungsgemäßen Spendervorrichtung 1. Diese Spendervorrichtung 1 weist dabei den beschriebenen Griffteil 100 und einen alternativ daran angekoppelten Spenderkopf 14 auf. Der Spenderkopf 14 weist bevorzugt ebenfalls eine Öffnung 9 zum Einfüllen einer fließfähigen Substanz in den Innenraum 24 auf. Der Spenderkopf 14 erstreckt sich dabei im Bereich 28 des zweiten Endes 27 in Tiefenrichtung T weiter als in Breitenrichtung B. Ferner erstreckt sich der Spenderkopf 14 im Bereich 26 des ersten Endes 25 in Tiefenrichtung T bevorzugt ebenfalls weiter als in Breitenrichtung B.

Der Spenderkopf 14 weist bevorzugt eine Vielzahl an Austrittsöffnungen 30 auf. Bevorzugt die die Anzahl der Austrittsöffnungen über die Länge des mit Austrittsöffnungen versehen Bereichs homogen verteilt. Es ist alternativ jedoch möglich, dass im Bereich 28 des zweiten Endes 27 mehr oder weniger Austrittsöffnungen 30 vorgesehen sind als in einem dazu beabstandeten bzw. weiter zum zweiten Ende 27 entfernten Bereich. Weiterhin ist es möglich, dass die Austrittsöffnungen 30 gleichförmig ausgebildet sind. Alternativ ist es jedoch möglich, dass die Austrittsöffnungen 30 im Bereich 28 des zweiten Endes 27 größer oder kleiner sind als Austrittsöffnungen 30, die zum zweiten Ende 27 weiter beabstandet sind. Zusätzlich oder alternativ können die Austrittsöffnungen 30 im Bereich 28 des zweiten Endes 27 eine andere Form aufweisen als Austrittsöffnungen 30, die zum zweiten Ende 27 weiter beabstandet sind.

Ferner lässt sich den Figuren 3b bis 3d entnehmen, dass die Oberfläche des Spenderkopfes 14 Erhebungen oder Noppen 32 aufweist. Bevorzugt weist der Spenderkopf 14 eine Vielzahl, insbesondere mindestens oder genau oder bis zu 5 Erhebungen oder Noppen 32 oder mindestens oder genau oder bis zu 10 Erhebungen oder Noppen 32 oder mindestens oder genau oder bis zu 15 Erhebungen oder Noppen 32 oder mindestens oder genau oder bis zu 20 Erhebungen oder Noppen 32 oder mindestens oder genau oder bis zu 30 Erhebungen oder Noppen 32 oder mindestens oder genau oder bis zu 50 Erhebungen oder Noppen 32 oder mindestens oder genau oder bis zu 10 Erhebungen oder Noppen 32 auf. Bevorzugt ist die Anzahl der Erhebungen oder Noppen 32 kleiner als die Anzahl der Austrittsöffnungen 30, insbesondere gibt es bevorzugt mindesten oder genau 1,1 mal oder mindesten oder genau 1,5 mal oder mindesten oder genau 2 mal oder mindesten oder genau 2,5 mal oder mindesten oder genau 3 mal mehr Austrittsöffnungen 30 als Erhebungen oder Noppen 30. Bevorzugt sind die Erhebungen oder Noppen 30 ausschließlich im Bereich 28 des zweiten Endes 27 ausgebildet oder sind bevorzugt in einem Bereich zwischen dem zweiten Ende 27 und 50% der Länge des Spenderkopfes 14 ausgebildet. Weiterhin sind bevorzugt in dem Bereich 28 des zweiten Endes 27 Austrittsöffnungen 30 und Noppen 32 angeordnet und in einem dazu beabstandeten oder benachbarten Bereich, der näher zum ersten Ende 25 beabstandet ist, sind bevorzugt nur Erhebungen / Noppen 32 oder Austrittsöffnungen 30 angeordnet.

Weiterhin ist es erfindungsgemäß möglich, dass anstelle der Erhebungen oder Noppen 32 oder zusätzlich dazu eine oder mehrere Vertiefungen, insbesondere Rillen, ausgebildet sein können.

Fig. 3c illustriert eine Draufsicht auf den erfindungsgemäßen Spenderkopf 14.

Fig. 3d kennzeichnet einen Schnitt B-B entlang der Spendervorrichtung 1 und durch Fig. 3e wird die sich aus dem Schnitt B-B ergebende Schnittdarstellung wiedergegeben.

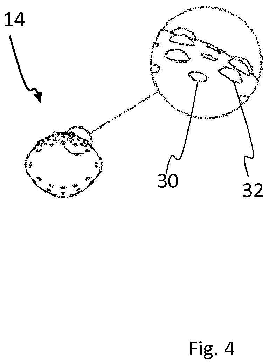

Fig. 4 zeigt eine Detaildarstellung eines Oberflächenbereichs des Spenderkopfes 14. Es ist dabei zu erkennen, dass die Noppen 32 bevorzugt eine gebogene Oberfläche aufweisen. Bevorzugt erheben sich die Noppen 32 um mindestens oder bis zu oder genau 0,1mm oder um mindestens oder bis zu oder genau 0,3mm oder um mindestens oder bis zu oder genau 0,5mm oder um mindestens oder bis zu oder genau 0,7mm oder um mindestens oder bis zu oder genau 1mm oder um mindestens oder bis zu oder genau 1,2mm oder um mindestens oder bis zu oder genau 1,3mm oder um mindestens oder bis zu oder genau 1,5mm oder um mindestens oder bis zu oder genau 2mm oder um mindestens oder bis zu oder genau 2,5mm oder um mindestens oder bis zu oder genau 3mm oder um mindestens oder bis zu oder genau 5mm gegenüber der umliegenden Oberfläche 34.

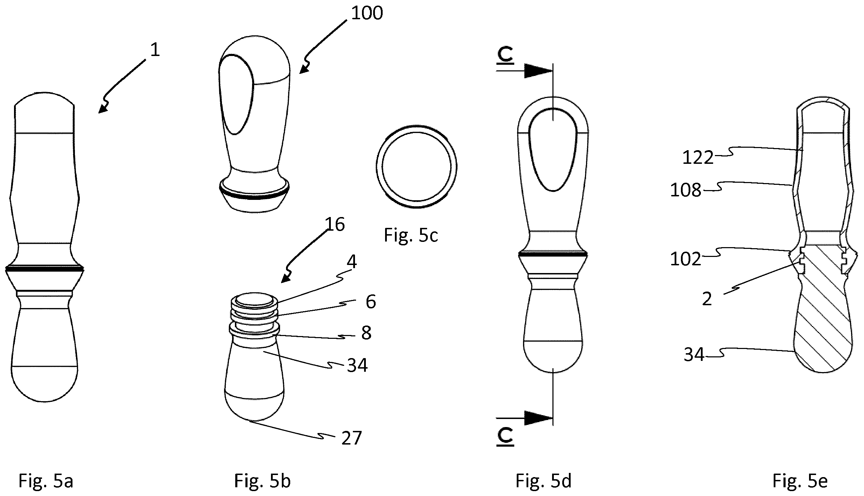

Die Figuren 5a bis 5e zeigen eine erfindungsgemäße Ausführungsform der Spendervorrichtung 1, gemäß welcher der Spenderkopf 16 bevorzugt teilweise oder mehrheitlich (volumenmäßig oder gewichtsmäßig) oder vollständig aus einem saugfähigen Material besteht. Diese Spendervorrichtung 1 weist dabei bevorzugt den beschriebenen Griffteil 100 und einen alternativ daran angekoppelten Spenderkopf 16 auf. Das saugfähige Material ist dabei bevorzugt ein Textil oder ein Schwamm, insbesondere ein Konjakschwamm. Analog zu den anderen Spenderköpfen 10, 12, 14 weist der Spenderkopf 16 bevorzugt ebenfalls eine Kopplungsstelle 2 und/oder einen Kragen 8 auf. Es ist hierbei möglich, dass der Spenderkopf 16 keinen definiert geformten Innenraum 24 aufweist oder ausbildet. Gemäß einer besonders bevorzugten Ausführungsform der vorliegenden Erfindung weist der Spenderkopf 16 jedoch einen definiert geformten oder definiert hergestellten Innenraum 24 bzw. Aufnahmeraum mit zugehöriger Öffnung auf.

Der Spenderkopf 14 weist über seine Oberfläche 34 verteilt eine Vielzahl an miteinander kommunizierend verbundener Poren auf. Der Spenderkopf 14 erstreckt sich bevorzugt im Bereich 28 des zweiten Endes 27 in Breitenrichtung B weiter als im Bereich 26 des ersten Endes 25.

Fig. 5c illustriert eine Draufsicht auf den erfindungsgemäßen Spenderkopf 16.

Fig. 5d kennzeichnet einen Schnitt C-C entlang der Spendervorrichtung 1 und durch Fig. 5e wird die sich aus dem Schnitt C-C ergebende Schnittdarstellung wiedergegeben.

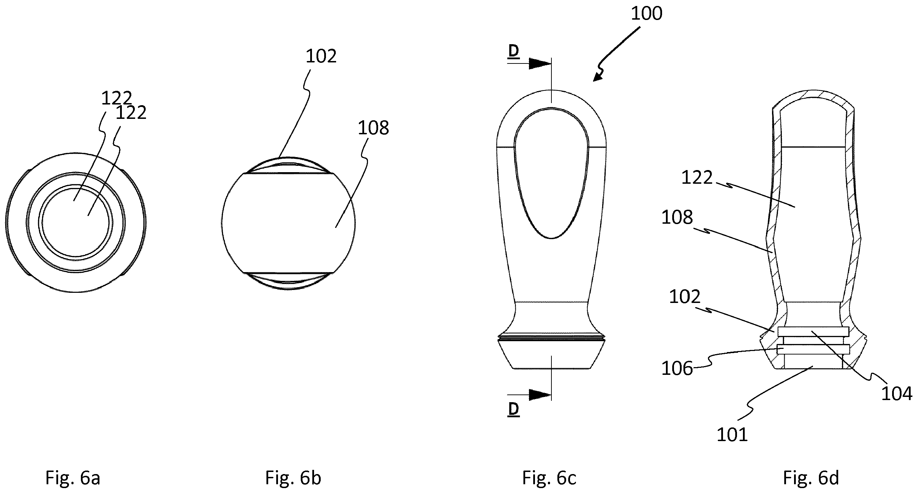

Die Fig. 6a-6d zeigen weitere Darstellungen des bereits beschriebenen Griffteils 100.

Die Fig. 6a zeigt eine Ansicht von unten auf den Griffteil 100. Gemäß dieser Ansicht ist die Griffteilöffnung 101 erkennbar, durch die hindurch der Innenraum 122 des Griffteils 100 erkennbar ist.

Fig. 6b zeigt eine Draufsicht von oben auf dem Griffteil 100. Es ist hierbei die äußere Oberfläche der Wandung 108 und ein Teil der die Griffkopplungsstelle 102 bildenden Wandung dargestellt.

Fig. 6d illustriert eine Draufsicht auf den erfindungsgemäßen Spenderkopf 16.

Fig. 6c kennzeichnet einen Schnitt D-D entlang der Spendervorrichtung 1 und durch Fig. 6d wird die sich aus dem Schnitt D-D ergebende Schnittdarstellung wiedergegeben.

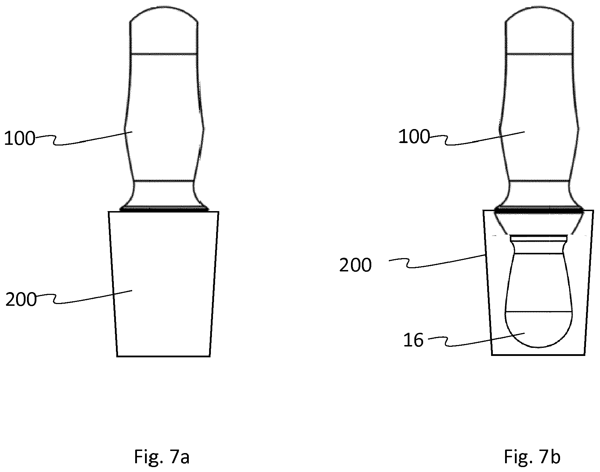

Fig. 7a zeigt eine erfindungsgemäße Spendervorrichtung 1, wobei der Spenderkopf 10, 12, 14, 16 der Spendervorrichtung 1 in eine Aufnahmeeinrichtung 200 eingesteckt oder eingeführt oder eingetaucht ist. Die Aufnahmeeinrichtung 200 dient dabei zum Bereitstellen von fließfähigen Substanzen und/oder zum Abdecken bzw. Schützen des Spenderkopfes 10, 12, 14, 16. Die Aufnahmeeinrichtung 200 kann dabei als Becher oder Kappe ausgebildet sein. Bevorzugt besteht die Aufnahmeeinrichtung 200 aus einem Polymermaterial oder Glas oder einem Keramikmaterial oder Metall oder einer Kombination mehrerer dieser Materialien oder weist eines oder mehrere dieser Materialien auf.

Fig. 7b zeigt eine Schnittdarstellung der Fig. 7a. Gemäß dieser Darstellung ist rein exemplarisch der Spenderkopf 16 in der Aufnahmeeinrichtung 200 angeordnet.

Bevorzugt bilden das Griffteil 100, ein oder mehrere Spenderköpfe 10, 12, 14, 16 und eine Aufnahmeeinrichtung 200 ein Set aus.

Die vorliegende Erfindung bezieht sich besonders bevorzugt auf Spenderkopf 14 zum Einbringen von fließfähigen Substanzen in einen Mund eines Lebewesens. Dieser Spenderkopf weist dabei bevorzugt mindestens auf: Einen sich länglich erstreckenden Grundkörper, wobei der Grundkörper in Längserstreckungsrichtung (L) einerseits ein erstes Ende 25 ausbildet und andererseits ein zweites Ende 27 ausbildet,
eine Kopplungsstelle 2 zum wiederholbaren und lösbaren Ankoppeln an einem Handteil 100, wobei die Kopplungsstelle 2 im Bereich des ersten Endes 25 des Grundkörpers angeordnet oder ausgebildet ist,
wobei der Grundkörper ein elastisch verformbares Material aufweist oder daraus besteht, insbesondere zumindest mehrheitlich daraus besteht,
wobei sich der Grundkörper in Tiefenrichtung (T) im Bereich 28 des zweiten Endes 27 gegenüber der Erstreckung in Tiefenrichtung (T) im Bereich 26 des ersten Endes 25 weiter erstreckt und bevorzugt im Bereich des zweiten Endes 27 die Wandung des Grundkörpers eine homogene Dicke aufweist, wobei der Grundkörper einen Innenraum 24 zum Aufnehmen von, fließfähigen Substanzen ausbildet, wobei der Grundkörper im Bereich der Kopplungsstelle 2 eine Zuführöffnung 9 zum Zuführen der fließfähigen Substanzen in den Innenraum 24 aufweist,
wobei in einer den Innenraum 24 des Grundkörpers begrenzenden Wandung 18 mehrere, definierte Austrittsöffnungen 30 angeordnet sind,
wobei die Austrittsöffnungen 30 jeweils einen Verschlussteil aufweisen, wobei der Verschlussteil infolge einer über die äußere Oberfläche einleitbaren

Unterdruckbeaufschlagung oder infolge einer über die innere Oberfläche einleitbaren Überdruckbeaufschlagung die Austrittsöffnung 30 öffnet.

Alternativ kann sich die vorliegende Erfindung auf einen Spenderkopf 10, 12 zum Einbringen von fließfähigen Substanzen in einen Mund eines Lebewesens beziehen, welcher mindestens aufweist: Einen sich länglich erstreckenden Grundkörper, wobei der Grundkörper in Längserstreckungsrichtung (L) einerseits ein erstes Ende 25 ausbildet und andererseits ein zweites Ende 27 ausbildet, eine Kopplungsstelle 2 zum wiederholbaren und lösbaren Ankoppeln an einem Handteil 100, wobei die Kopplungsstelle 2 im Bereich 26 des ersten Endes 25 des Grundkörpers angeordnet oder ausgebildet ist, wobei der Grundkörper ein elastisch verformbares Material aufweist und elastisch verformbare Lamellen 20 ausbildet. Bevorzugt bilden die Lamellen 20 mehr als 50% der Gesamtoberfläche des Grundkörpers aus und/oder verändert sich die Höhe mindestens einer Lamelle 20 in Abhängigkeit vom Abstand zum zweiten Ende 27 des Grundkörpers.

Bevorzugt erstrecken sich die Lamellen 20 in ihrer Längserstreckungsrichtung zwischen dem ersten Ende 25 und dem zweiten Ende 27 des Grundkörpers erstrecken, insbesondere einen ersten Bereich 26 um das erste Ende 25 mit einem zweiten Bereich 28 um das zweite Ende 27 verbinden, wobei sich der erste Bereich 26 bis zu 45%, insbesondere bis zu 30% oder bis zu 20%, der Länge des Grundkörpers vom ersten Ende 25 aus in Längsrichtung des Grundkörpers zum zweiten Ende 27 hin erstreckt und sich der zweite Bereich 28 bis zu 30%, insbesondere bis zu 20% oder bis zu 15% oder bis zu 10% oder bis zu 5%, der Länge des Grundkörpers vom zweiten Ende 27 aus in Längsrichtung des Grundkörpers zum ersten Ende 25 hin erstreckt.

Bevorzugt erstrecken sich mehrere Lamellen 20 oder alle Lamellen 20 zumindest abschnittsweise spiralförmig.

Bevorzugt sind die Lamellen 20 in einem Winkel zwischen 45° und 135°, insbesondere von 90° oder im Wesentlichen 90° oder genau 90°, gegenüber der Längserstreckungsrichtung L des Grundkörpers ausgerichtet.

Besonders bevorzugt sind die Lamellen 20 scheibenförmig oder schalenförmig ausgebildet und weisen bevorzugt eine umlaufende Außenkante auf, die abschnittsweise oder vollständig eine runde oder elliptische Linie beschreibt, wobei die umlaufende Außenkante bei zumindest einer ersten Lamelle 20 größer ist wie bei einer zweiten Lamelle 20, wobei die erste Lamelle 20 näher zum ersten Ende 25 hin angeordnet ist als zum zweiten Ende 27 und die zweite Lamelle 20 näher zum zweiten Ende hin angeordnet ist als zum ersten Ende.

**Bezugszeichenliste:**

| | | | |
|---|---|---|---|
| 1 | Spendervorrichtung | 9 | Öffnung |
| 2 | Kopplungsstelle | 10 | Spenderkopf mit spiralförmigen Lamellen |
| 4 | erstes Formschlusselement | 12 | Spenderkopf mit scheibenförmigen Lamellen |
| 6 | zweites Formschlusselement | | |
| 8 | Kragen | | |
| 14 | Spenderkopf mit breiterem zweiten Endbereich | 100 | Griffteil |
| 16 | Spenderkopf aus saugfähigem Material | 101 | Griffteilöffnung |
| 18 | Wandung | 102 | Griffkopplungsstelle |
| 20 | Lamelle | 104 | erstes Gegenstück |
| 22 | Verschlusseinrichtung | 106 | zweites Gegenstück |
| 24 | Aufnahmeraum / Innenraum des Spenderkopfes | 108 | Wandung |
| 25 | erstes Ende | 122 | Innenraum des Griffteils |
| 26 | Bereich des ersten Endes | 200 | Kappe / Becher / Aufnahmeeinrichtung |
| 27 | zweites Ende | B | Breitenrichtung |
| 28 | Bereich des zweiten Endes | L | Längsrichtung |
| 30 | Austrittsöffnungen | R | Radius |
| 32 | Erhebungen / Noppen | r | radiale Richtung |
| 34 | umliegende Oberfläche | T | Tiefenrichtung |

## Patentansprüche

1. Spenderkopf (14) zum Einbringen von fließfähigen Substanzen in einen Mund eines Lebewesens,
mindestens umfassend
ein sich länglich erstreckender Grundkörper, wobei der Grundkörper in Längserstreckungsrichtung (L) einerseits ein erstes Ende (25) ausbildet und andererseits ein zweites Ende (27) ausbildet,
eine Kopplungsstelle (2) zum wiederholbaren und lösbaren Ankoppeln an einem Handteil (100), wobei die Kopplungsstelle (2) im Bereich des ersten Endes (25) des Grundkörpers angeordnet oder ausgebildet ist,
wobei der Grundkörper ein elastisch verformbares Material aufweist,
wobei sich der Grundkörper in Tiefenrichtung (T) im Bereich (28) des zweiten Endes (27) gegenüber der Erstreckung in Tiefenrichtung (T) im Bereich (26) des ersten Endes (25) weiter erstreckt und bevorzugt im Bereich des zweiten Endes (27) die Wandung des Grundkörpers eine homogene Dicke aufweist, wobei
der Grundkörper einen Innenraum (24) zum Aufnehmen von, fließfähigen Substanzen, insbesondere Nahrung und/oder Medikamenten und/oder Getränken und/oder Gels, ausbildet, wobei der Grundkörper im Bereich der Kopplungsstelle (2) eine Zuführöffnung (9) zum Zuführen der fließfähigen Substanzen in den Innenraum (24) aufweist, wobei in einer den Innenraum (24) des Grundkörpers begrenzenden Wandung (18) mehrere,
insbesondere mindestens 2 oder mindestens oder genau oder bis zu 5 oder mindestens oder genau oder bis zu 10 oder mindestens oder genau oder bis zu 20 oder mindestens oder genau oder bis zu 50, definierte Austrittsöffnungen (30) angeordnet sind, wobei
die Austrittsöffnungen (30) jeweils einen Verschlussteil aufweisen, wobei der Verschlussteil infolge einer über die äußere Oberfläche einleitbaren Unterdruckbeaufschlagung oder infolge einer über die innere Oberfläche einleitbaren Überdruckbeaufschlagung die Austrittsöffnung (30) öffnet.

2. Spenderkopf nach Anspruch 1,
**dadurch gekennzeichnet, dass**
im Innenraum (24) ein Schwamm, insbesondere auswechselbar, angeordnet ist und/oder
die äußere Oberfläche (34) des Grundkörpers eine Vielzahl an Erhebungen (32) und/oder Vertiefungen, insbesondere Noppen und/oder Rillen, insbesondere Längsrillen und/oder Querrillen, und/oder Lamellen (20), insbesondere Längslamellen und/oder Querlamellen, zum Reinigen der Zunge und/oder der Schleimhäute aufweist.

3. Spenderkopf (16) zum Einbringen von fließfähigen Substanzen in einen Mund eines Lebewesens,
mindestens umfassend
ein sich länglich erstreckender Grundkörper, wobei der Grundkörper in Längserstreckungsrichtung einerseits ein erstes Ende (25) ausbildet und andererseits ein zweites Ende (27) ausbildet,
eine Kopplungsstelle (2) zum wiederholbaren und lösbaren Ankoppeln an einem Handteil (100), wobei die Kopplungsstelle (2) im Bereich (26) des ersten Endes (25) des Grundkörpers angeordnet oder ausgebildet ist,
wobei der Grundkörper durch einen definiert geformte Schwamm, insbesondere einen Konjakschwamm, ausgebildet wird, wobei die Kopplungsstelle (2) zur definierten reib- und/oder kraftschlüssigen Kopplung mit einem Verbindungsteils, insbesondere eines Griffteils, aufbereitet ist.

4. Spenderkopf (14, 16) nach einem der vorangegangenen Ansprüche,
**dadurch gekennzeichnet, dass**
im Bereich des ersten Endes (25) des Grundkörpers ein umlaufender Kragen (8) ausgebildet ist, wobei sich der Kragen (8) in radialer Richtung (r) weiter erstreckt als alle anderen Bestandteile des Spenderkopfs (14, 16).

5. Griffteil (100) zur Ankopplung an einen Spenderkopf (14, 16) gemäß einem der vorangegangenen Ansprüche,
mindestens aufweisend
einen sich bevorzugt länglich erstreckenden Basiskörper,
wobei der Basiskörper einen elastisch verformbaren Aufnahmeraum (122) zum Aufnehmen einer fließfähigen Substanz, insbesondere einer Flüssigkeit, und eine Öffnung (101) zum Zuführen und Ausgeben der fließfähigen Substanz ausbildet, wobei das Volumen des Aufnahmeraums (122) infolge einer elastischen Verformung veränderbar ist,
wobei die Öffnung (101) im Bereich einer Anbringungseinrichtung (102) zur Ankopplung des Spenderkopfs (14, 16) ausgebildet ist,
wobei die Anbringungseinrichtung (102) zum, insbesondere kraftschlüssigen und/oder formschlüssigen, Zusammenwirken mit einem an einem Spenderkopf (14, 16) angeordneten Verbindungsteil oder einer Kopplungsstelle (2) des Spenderkopfs (14, 16) ausgebildet ist.

6. Set
zumindest bestehend
aus einem Griffteil (100) nach Anspruch 14 und einem Spenderkopf (14, 16) nach einem der Ansprüche 1 bis 13 und bevorzugt aus einem Aufnahmebehältnis (200) zum Vorhalten einer Flüssigkeit und zum Einbringen des Spenderkopfs (14, 16),
wobei der Aufnahmeraum (122) des Griffteils (100) und Spenderkopf, insbesondere der Innenraum des Spenderkopfes (14, 16), in einer Gebrauchskonfiguration eine Fluidverbindung ausbilden.
